# EUROPEAN PATENT SPECIFICATION

(11) **EP 3 981 422 B1**
(45) Date of publication and mention of the grant of the patent: **01.05.2024**
(21) Application number: 20843577.6
(22) Date of filing: 29.05.2020
(51) Int. Cl.: A61K 38/47, A61P 17/06

(54) **PHARMACEUTICAL COMPOSITION CONTAINING LYSOZYME AND USE THEREOF**
PHARMAZEUTISCHE ZUSAMMENSETZUNG MIT LYSOZYM UND VERWENDUNG DAVON
COMPOSITION PHARMACEUTIQUE CONTENANT DU LYSOZYME ET SON UTILISATION

(30) Priority: 19.07.2019 CN 201910656136
(43) Date of publication of application: 13.04.2022
(73) Proprietor: Guangzhou Century Clinical Research Co., Ltd, Guangzhou, Guangdong 510530 (CN); Guangzhou Xin-Chuangyi Biopharmaceutical Co., Ltd, Huangpu District Guangzhou, Guangdong 510535 (CN); Guangzhou Welman New Drug R&D Co,. Ltd., Guangzhou, Guangdong 510620 (CN); Xiangbei Welman Pharmaceutical Co., Ltd, Hunan 410331 (CN); Nanjing Kangfushun Pharmaceutical Co., Ltd, Nanjing, Jiangsu 210046 (CN)
(72) Inventor: SUN, Mingjie, Guangzhou, Guangdong 510620 (CN); SUN, Tianyu, Guangzhou, Guangdong 510620 (CN)
(74) Representative: Kraus & Lederer PartGmbB
(86) International application number: PCT/CN2020/093314
(87) International publication number: WO 2021/012789

(56) References cited:
- WO-A2-2010/124280
- CN-A- 1 548 152
- CN-A- 1 557 485
- CN-A- 1 593 652
- CN-A- 1 663 610
- CN-A- 102 108 346
- CN-A- 105 561 302
- CN-A- 105 770 869
- CN-A- 109 646 412
- KOH D. et al. .: "Salivary Immunoglobulin A and Lysozyme in Patients with Psoriasis.", Annals of the Academy of Medicine., vol. 33, no. 3, 31 May 2004 (2004-05-31), XP055775155,
- Xia Yingkui: "The value of total complement , C3 and lysozyme in psoriasis patient and current research direction of mechanism of psoriasis", Journal of China Medical University, vol. 13, no. 3, 31 December 1984 (1984-12-31), pages 70-74, XP055878026,

## Description

### TECHNICAL FIELD

The present disclosure relates to the field of medicine, in particular to a pharmaceutical composition containing lysozyme and the use thereof, especially in the prevention of psoriasis, treatment of psoriasis, and/or prevention of psoriasis recurrence.

### BACKGROUND

Psoriasis is a common chronic skin disease. According to the pathological characteristics, psoriasis can be roughly divided into plaque psoriasis, guttate psoriasis, pustular psoriasis, erythrodermic psoriasis, etc. Plaque psoriasis is the most common, accounting for not less than 90% of psoriasis, and is mainly characterized by red, raised skin covered with silvery white scales. Guttate psoriasis is caused by bacterial infection and is characterized by small drop-like erythema on the skin. Pustular psoriasis is mainly characterized by sterile pustules on the skin. Erythrodermic psoriasis is mainly characterized by diffuse inflammatory erythema and skin scales on a large area of the skin and is often accompanied by fever or even high fever. Psoriasis may also lead to other complications, such as arthritis, cardiovascular diseases, metabolic disorders, and malignant tumors. The incidence of psoriasis worldwide has reached 2-3%, which has seriously affected the quality of life of hundreds of millions of patients.

There are two main drug therapies for psoriasis: local treatment and systemic treatment, mainly through local resistance to cell proliferation or suppression of immune response to improve skin symptoms. Local treatment is the most commonly used method, and corticosteroids, vitamin D analogs, tretinoin analogs, etc. are all widely used drugs. They may often be effective for incipient psoriasis or mild psoriasis, and topical use of the drugs is convenient and readily accepted by patients. In addition, for guttate psoriasis, since the main cause thereof is bacterial infection, topical bactericides or topical antibacterial drugs can usually be therapeutically effective. However, topical drugs can only be used for local symptomatic treatment and are less effective for psoriasis with a relatively wide range of systemic involvement. Systemic treatment is mainly for moderate to severe psoriasis, and, for example, oral administration or injection of immunosuppressants such as methotrexate and cyclosporine, or injection of targeted biological preparations are commonly used. Systemic treatment can regulate systemic immunity and has a relatively strong effect. However, the systemic use of an immunosuppressant is more toxic and difficult to be tolerated by patients for a long time; and biological preparations require injection, have a single target of action, are expensive, and critically, usually lead to more rapid recurrence of the disease.

Clinically, there are many patients with psoriasis who are resistant to existing drugs. Some patients themselves are very poorly responsive to existing drugs, which makes it difficult to relieve the disease; for some patients, the use of drugs induces the disease to become conversely more serious; and some patients relapse soon after using existing drugs to relieve the condition of the disease, and the condition of recurrence is more serious, so it is difficult to obtain clinical benefits by continuing to use the previous therapies. For such patients, there is an urgent need to find a more effective therapy.

CN 1557485 A discloses the use of recombinant human lysozyme as a preparation drug for treating inflammation caused by pathogenic microorganism infection. The inflammation includes skin inflammation, which lists psoriasis. For skin inflammation, it specify that the drug form for treating skin inflammation is injection. There is no disclosed technical solution for the treatment of psoriasis with oral preparations.

CN 1593652 A discloses the use of human lysozyme in the preparation of drugs for treating skin diseases. It discloses the use of human lysozyme in the preparation of drugs for treating psoriasis caused by immune diseases of the human body. However, oral preparations are not involved, and they are all for external use.

CN 105561302 A discloses a pharmaceutical composition for treating psoriasis, wherein the active components contain lysozyme and danshensu. All of them are for external use, and the effect of lysozyme alone is not good.

CN 105770869 A discloses a pharmaceutical composition for treating psoriasis, wherein the active components contain lysozyme and emodin; all of them are for external use, and the effect of lysozyme alone is not good.

### SUMMARY

The invention is set out in the appended set of claims.

An object of the present disclosure is the use of lysozyme in the preparation of a medicament for preventing psoriasis, treating psoriasis, and/or preventing psoriasis recurrence.

Another object of the present disclosure is to provide a pharmaceutical composition for preventing psoriasis, treating psoriasis, and/or preventing psoriasis recurrence.

The technical solutions used by the present disclosure are as follows:
Disclosed is a use of lysozyme in the preparation of a medicament for preventing psoriasis, treating psoriasis, and/or preventing psoriasis recurrence. The psoriasis can be any type of psoriasis, including but not limited to psoriasis vulgaris, pustular psoriasis or erythrodermic psoriasis; and the PASI score of the psoriasis is not less than 5. The lysozyme is prepared into a medicament, and the medicament is an enteric preparation.

In some embodiments of the use, the PASI score of the psoriasis is not less than 8.

In some embodiments of the use, the psoriasis is drug-resistant psoriasis.

In some embodiments of the use, for the drug-resistant psoriasis, treatments with anti-inflammatory agents, anti-cell proliferation agents, and/or immunomodulators have been found ineffective.

In some embodiments of the use, for the drug-resistant psoriasis, treatments with at least one selected from the group consisting of corticosteroids, vitamin D analogs, tretinoin analogs, calcineurin inhibitors, anthralin, coal tar, salicylic acid, methotrexate, phosphodiesterase inhibitors, aryl hydrocarbon receptor agonists, Janus kinase inhibitors, tumor necrosis factor inhibitors and interleukin inhibitors have been found ineffective.

In some embodiments of the use, the medicament is an enteric preparation, which is a preparation that releases the drug in the small intestine or the large intestine. In particular, the small intestine is duodenum, jejunum, and/or ileum, and the large intestine is cecum, colon, and/or rectum.

In some embodiments of the use, the enteric preparation is a preparation that releases the drug in the large intestine, especially a preparation that releases the drug in the colon, i.e., a colonic enteric- preparation.

In some embodiments of the use, the medicament can be in any dosage form from enteric tablets, enteric capsules, enteric soft capsules, enteric pills, enteric pellets, enteric dripping pills, enteric granules, enteric nanoparticles, enteric sustained-release preparations, and enteric controlled-release preparations.

In some embodiments of the use, the medicament further comprises a pharmaceutically applicable excipient, including but not limited to an enteric matrix material, an enteric coating material, a diluent, a filler, a lubricant, a disintegrant, a glidant, an absorption enhancer, or a flavoring agent.

In some embodiments of the use, the potency of the lysozyme in the medicament is more than 20000 U/mg.

In some embodiments of the use, the dosage of the lysozyme in the medicament is 0.3-20 g/day.

In some embodiments of the use, the dosage of the lysozyme in the medicament is 0.5-15 g/day.

In some embodiments of the use, the dosage of the lysozyme in the medicament is 1-6 g/day.

Further, the medicament can be administered in divided doses, 1-3 times a day.

Disclosed is a pharmaceutical composition for preventing or treating a disease, wherein the composition comprises lysozyme; preventing or treating the disease refers to preventing psoriasis, treating psoriasis, and/or preventing psoriasis recurrence; and the psoriasis can be any type of psoriasis, including but not limited to psoriasis vulgaris, pustular psoriasis or erythrodermic psoriasis.

In some embodiments of the pharmaceutical composition, the PASI score of the psoriasis is not less than 5.

In some embodiments of the pharmaceutical composition, the PASI score of the psoriasis is not less than 8.

In some embodiments of the pharmaceutical composition, the psoriasis is drug-resistant psoriasis.

In some embodiments of the pharmaceutical composition, for the drug-resistant psoriasis, treatments with anti-inflammatory agents, anti-cell proliferation agents, and/or immunomodulators have been found ineffective.

In some embodiments of the pharmaceutical composition, for the drug-resistant psoriasis, treatments with at least one selected from the group consisting of corticosteroids, vitamin D analogs, tretinoin analogs, calcineurin inhibitors, anthralin, coal tar, salicylic acid, methotrexate, phosphodiesterase inhibitors, aryl hydrocarbon receptor agonists, Janus kinase inhibitors, tumor necrosis factor inhibitors and interleukin inhibitors have been found ineffective.

In some embodiments of the pharmaceutical composition, the pharmaceutical composition is an oral preparation.

In some embodiments of the pharmaceutical composition, the oral preparation is an enteric preparation, which is a preparation that releases the drug in the small intestine or the large intestine. In particular, the small intestine is duodenum, jejunum, and/or ileum, and the large intestine is cecum, colon, and/or rectum.

In some embodiments of the pharmaceutical composition, the enteric preparation is a preparation that releases the drug in the large intestine, especially a preparation that releases the drug in the colon, i.e., a colonic enteric preparation.

In some embodiments of the pharmaceutical composition, the pharmaceutical composition is in any dosage form from enteric tablets, enteric capsules, enteric soft capsules, enteric pills, enteric pellets, enteric dripping pills, enteric granules, enteric nanoparticles, enteric sustained-release preparations, and enteric controlled-release preparations.

In some embodiments of the pharmaceutical composition, the pharmaceutical composition further comprises a pharmaceutically applicable excipient, including but not limited to an enteric matrix material, an enteric coating material, a diluent, a filler, a lubricant, a disintegrant, a glidant, an absorption enhancer, or a flavoring agent.

In some embodiments of the pharmaceutical composition, the potency of the lysozyme in the pharmaceutical composition is more than 20000 U/mg.

In some embodiments of the pharmaceutical composition, the dosage of the lysozyme in the pharmaceutical composition is 0.3-20 g/day.

In some embodiments of the pharmaceutical composition, the dosage of the lysozyme in the pharmaceutical composition is 0.5-15 g/day.

In some embodiments of the pharmaceutical composition, the dosage of the lysozyme in the pharmaceutical composition is 1-6 g/day.

Furthermore, after the dosage of the drug is determined, the drug is administered 1-3 times a day.

Beneficial Effects of the disclosure:
The applicant has unexpectedly discovered that lysozyme has excellent clinical effects on the treatment or prevention of drug-resistant psoriasis, and compared with the use of traditional oral drugs, immunosuppressants, biological preparations, etc., the time before the recurrence of the disease can be significantly prolonged and the recurrence rate of the disease can be significantly reduced. Since lysozyme itself is a substance with good safety, the method provided by the present disclosure is very suitable for children, pregnant women, and patients with drug-resistant psoriasis who have low immunity or relatively poor hepatic and renal functions.

### DETAILED DESCRIPTION

The references to the methods of treatment by therapy or surgery or *in vivo* diagnosis methods in examples below of this description are to be interpreted as references to compounds, pharmaceutical compositions and medicaments of the present invention for use in those methods.

Embodiments are further listed below to illustrate the present disclosure in detail. It should also be understood that the following embodiments are only used to further illustrate the present disclosure and should not be construed as limiting the scope of the present disclosure, and some non-essential improvements and adjustments made by those skilled in the art based on the principles set forth in the present disclosure all fall within the scope of the present disclosure. The specific process parameters, etc. in the following embodiments are also only examples in the appropriate ranges, that is, those skilled in the art would have been able to make selections within the appropriate ranges according to the description herein, without limitation to the specific data exemplified hereinbelow.

### Definition:

The Chinese term Rongiunmei, i.e., lysozyme. Lysozyme of the present disclosure, can be lysozyme derived from animals, plants or microorganisms, or recombinant products of natural lysozyme. For example, it can be egg white lysozyme, human lysozyme, recombinant human lysozyme, phage lysozyme, etc. The lysozyme in the present disclosure includes pharmaceutically acceptable salts thereof, such as hydrochloride, chloride, sulfate, or amino acid salts. Lysozyme was first discovered by Fleming as an endogenous enzyme widely present in organisms. Lysozyme has been approved for edible or medicinal use worldwide. In the United States lysozyme has been Generally Recognized as Safe(GRAS). WHO, many European countries, Japan and China have approved it for use as a food additive. It has been approved for medicinal use in China, Japan, Singapore and other countries for the treatment of rhinitis, laryngopharyngitis, oral ulcers, chickenpox, herpes zoster, flat warts, etc. Products on the market include Neuzym^{®}, Mucozome^{®}, Laisuorui^{®}, etc. The mechanism of the antibacterial effect of lysozyme mainly involves hydrolyzing peptidoglycan in the cell wall of bacteria, and the mechanism of the antiviral effect mainly involves charge interaction with negatively charged viruses.

Enteric preparation: An enteric preparation refers to a preparation that does not release or hardly releases the drug in the stomach but can release most or all of the drug in certain parts of the intestine when entering the intestine. The human intestine includes the small intestine and the large intestine, wherein the small intestine is further divided into duodenum, jejunum, and ileum, and the large intestine is further divided into cecum, colon, and rectum. Different parts of the digestive tract have different pH values. For example, the pH value in the stomach is about 1-3, the pH value in the small intestine is about 4-7, and the pH value in the large intestine is about 7-8. By selecting a pH-dependent degradable material as a preparation excipient, it is possible to prepare a preparation that directionally releases the drug in a specific part of the digestive tract, e.g., a small intestine enteric preparation or a large intestine enteric preparation. In particular, the preparations include duodenal enteric preparations, jejunal enteric preparations, ileal enteric preparations, cecal enteric preparations, colonic enteric preparations, or rectal enteric preparations, etc.

The Chinese term Yinxiebing, i.e., psoriasis, can be judged according to standard medical diagnosis methods. Psoriasis can be classified in different ways. Psoriasis can be classified into types according to pathological characteristics, e.g., guttate psoriasis, psoriasis vulgaris, pustular psoriasis or erythrodermic psoriasis, etc. The specific distinguishing methods are well known in the field of medical diagnosis. Psoriasis can be divided into mild, moderate, and severe according to the severity. The severity can be determined by PASI (Psoriasis Area Severity Index) scores. PASI is a set of scoring indicators that are widely used internationally to reflect the severity of psoriasis. In the scoring system, four parts, i.e., the head, upper limbs, torso, and lower limbs, are used for comprehensive scoring according to indicators such as erythema, desquamation, infiltration, and area of involvement. The higher the score, the more severe the condition of psoriasis. The specific scoring rules are well known in the field of medical diagnosis. In addition, according to the site of the disease, psoriasis can also be divided into scalp psoriasis, intertriginous psoriasis, nail/toenail psoriasis, etc.

Drug-resistant psoriasis refers to psoriasis that is resistant to certain drugs, and these drugs have been found ineffective for such psoriasis after being administered to the subject. These drugs include topical treatment drugs and systemic treatment drugs, wherein the topical treatment drugs include, but are not limited to, corticosteroids, vitamin D analogs, tretinoin analogs, calcineurin inhibitors, aryl hydrocarbon receptor agonists, anthralin, coal tar, salicylic acid, and other drugs suitable for locally exerting a therapeutic effect on psoriasis, and the systemic treatment drugs include, but are not limited to, tretinoin analogs, methotrexate, calcineurin inhibitors, phosphodiesterase inhibitors, Janus kinase inhibitors, tumor necrosis factor inhibitors, interleukin inhibitors, and other drugs suitable for systemically exerting a therapeutic effect on psoriasis.

Effective: The PASI score of psoriasis after treatment is less than or equal to 50% of the score before treatment, that is, the PASI score after treatment is reduced by 50% or more than 50%.

Ineffective: The PASI score of psoriasis after treatment is more than 50% of the score before treatment, namely, PASI after treatment ÷ PASI before treatment × 100% > 50%.

Recurrence: For patients who have been effectively treated, after the treatment is discontinued for a period of time, the PASI score rises again to not less than 50% of the PASI score before treatment.

In the present disclosure, imiquimod-induced psoriasis model animals were used to screen out individuals on which the topical drug effect was relatively poor, and low-dose imiquimod was used to maintain psoriatic lesions in the animals where the overall level of inflammatory factors was not high. They were treated with an ordinary oral lysozyme preparation and an enteric lysozyme preparation, respectively, and the results showed that the enteric preparation had better effects. It was observed that animal intestinal dendritic cells (especially colonic dendritic cells) treated with the different preparations showed a significant difference in quantity. In addition, immunoglobulin E related to hypersensitivity was significantly increased, and was consistent with the increasing trend of the quantity of intestinal dendritic cells. The results suggested that the excessive proliferation of intestinal dendritic cells and the excessive increase of the body sensitivity includes one of the reasons for the resistance of certain drug-resistant psoriasis to drugs; the mechanism of the enteric lysozyme preparation in treating drug-resistant psoriasis and preventing the recurrence of the disease is related to the inhibitory effect on intestinal dendritic cells; and for these drug-resistant psoriasis, simply adjusting the level of inflammatory factors according to conventional treatment methods can not achieve the effect of treatment and prevention of recurrence.

The present disclosure will be specifically illustrated below through animal experiments and cases.

The test animals, reagents etc. used were all commercially available. The relevant test methods used in the experiments, the specific operation methods of the relevant instruments, etc., were well known to those skilled in the art.

The lysozyme granules, lysozyme enteric granules, and enteric placebo granules used were homemade. The preparation method involved: taking lysozyme with a potency of more than 20000 U/mg as a raw material, adding the same amount of starch, mixing them until uniform, adding a binder (water) for granulation, and drying the granules to obtain lysozyme granules. The prepared lysozyme granules were coated with an enteric coating (HPMC) to obtain lysozyme enteric granules. Enteric placebo granules were prepared by the same process as that for the lysozyme enteric granules, except that no lysozyme was used and only starch was used as the raw material. The lysozyme buccal tablets used were "Xinrong Junmei" produced by Xiangbei Welman Pharmaceutical Co., Ltd., and were mainly prepared by using excipients for ordinary tablets and flavoring agents. The lysozyme enteric tablets used were "Laisuorui" produced by Xiangbei Welman Pharmaceutical Co., Ltd., and were mainly prepared by using excipients for ordinary tablets and an enteric coating material to facilitate the drug release mainly in the small intestine. The colonic enteric lysozyme tablets used were prepared by Xiangbei Welman Pharmaceutical Co., Ltd., by pressing lysozyme as the raw material and excipients for ordinary tablets into tablets and then coating the tablets with a commercially available colonic enteric coating material to facilitate the drug release mainly in the colon area.

### Example 1 Effects of lysozyme granules and lysozyme enteric granules on imiquimod-induced psoriasis-like animal model

Test drugs: Lysozyme granules, lysozyme enteric granules, and enteric placebo granules.

Test animals: BALB/C mice weighing 18-22 g.

Modeling: Imiquimod was used to induce psoriasis-like lesions on the skin of mice. The back of each mouse was depilated to form a bare skin area of about 3 cm × 2 cm, and the animals in a modeling group were smeared with about 60 mg of 5% imiquimod cream daily on the bare skin area, while the animals in a normal control group were smeared with an equal amount of Vaseline for 7 consecutive days. It was observed that on day 2, the skin of the animals in the modeling group showed erythema, and on days 3 and 4, scales appeared, accompanied by significantly thickened skin and gradually darkened erythema. These changes were very similar to the skin damage in human psoriasis; in addition, the levels of factors such as TNF-α and IL-17 were significantly higher than those of mice in the normal control group, indicating that the modeling was successful. With reference to the human PASI evaluation standards, a method for evaluating the severity of psoriasis in mice was established, which involved taking erythema, scales, and skin thickness as indicators, scoring from 0 to 4 according to the degree of symptoms, and adding the scores of the three indicators to obtain the total mPASI score. The scoring standards were: 0 point for asymptomatic, 1 point for mild, 2 points for moderate, 3 points for severe, and 4 points for extremely severe.

Screening: After 7 days of modeling, each successfully modeled mouse was scored by mPASI. Since discontinuation of imiquimod might cause the psoriasis-like symptoms of the mice to gradually decrease over time, they were still smeared with a 10 mg low-dose imiquimod cream daily for maintenance after the completion of the modeling. In addition, the lesion area was smeared with about 30 mg of a 0.005% calcipotriol ointment once a day for 3 consecutive days. After using calcipotriol for 3 days, animals whose mPASI scores were improved by less than 50% were screened out, and a total of 24 animals were used for the next step of operation.

Grouping and administration: The screened 24 mice were randomly divided into three groups: a lysozyme granule group, an lysozyme enteric granule group, and an enteric-coated placebo granule group, with 8 mice in each group. In addition, 8 mice from the normal control group at the modeling stage were used as a normal control group. Each group was given the corresponding drug, respectively. Animals in the lysozyme granule group were given lysozyme granules, animals in the lysozyme enteric granule group were given lysozyme enteric granules, and animals in the enteric placebo granule group and the normal control group were given the enteric placebo granules, at a dosage of 20 mg/kg, once a day, for 7 consecutive days. During this period, all groups except the normal control group continued to be smeared with the 5% imiquimod cream at a low dose of 10 mg daily. all the animals completed the experimental process except that one mouse died in the placebo group.

Result and, analysis: After the administration was completed, the survival mice were scored by mPASI, and the mPASI improvement rate compared to mPASI before the administration was calculated. mPASI improvement rate = (mPASI before administration - mPASI after administration) = mPASI before administration. Venous blood was taken from the mice, and the serum levels of TNF-α, IFN-γ, IL-17 and IgE were determined by enzyme-linked immunosorbent assay. After the animals were sacrificed, the small intestine and the colon were separated and respectively prepared into a small intestine single cell suspension and a colon single cell suspension, the small intestine single cell suspension and the colon single cell suspension were labeled with mouse CD11c monoclonal antibody, and the numbers (percentage) of dendritic cells in the small intestine single cell suspension and the colon single cell suspension were determined by means of flow cytometry, respectively. Tables 1-3 were results of experimental data after statistical analysis, wherein the symbol * indicated p < 0.05, compared with the placebo group, and the symbol ** indicated p < 0.01, compared with the placebo group; the symbol # indicated p < 0.05, compared with the ordinary granule group, and the symbol ## indicated p < 0.01, compared with the ordinary granule group; and the symbol & indicated p < 0.05, compared with the normal control group, and the symbol && indicated p < 0.01, compared with the normal control group.

**Table 1 mPASI improvement rate of psoriasis model mice after treatment**

| | **Number of mice** | **mPASI improvement rate (%)** |
|---|---|---|
| Normal control group | 8 | / |
| Enteric placebo granule group | 7 | -1.9±4.7 |
| Ordinary lysozyme granule group | 8 | 4.2±1.0 |
| ysozyme enteric granule group | 8 | **63.1±6.2**^{##}** |

**Table 2 Effects on serological indicators of psoriasis model mice after treatment**

| | **Number of mice** | **TNF-α (pg/ml)** | **IFN-γ (pg/ml)** | **IL-17 (pg/ml)** | **IgE (pg/ml)** |
|---|---|---|---|---|---|
| Normal control group | 8 | 276.13±23.36 | 65.74±7.55 | 129.53±10. 15 | 47.71±3.2 4 |
| Enteric placebo granule group | 7 | 313.60±11.92 ^{&&} | 71.30±3.79 | 148.56±8.1 2^{&} | 850.55±4 1.24^{&&} |
| Ordinary lysozyme granule group | 8 | 292.48±16.61 | 68.24±4.96 | 136.83±12. 52 | 826.95±2 8.25^{&&} |
| Lysozyme enteric granule group | 8 | 299.72±31.43 | 73.95±7.83^{&} | 156.30±15. 29^{&&##} | **60.43±4.6 6**^{##}** |

**Table 3 Effects on the number of intestinal dendritic cells in psoriasis model mice after treatment**

| | **Number of mice** | **Number of small intestinal dendritic cells (%)** | **Number of colon dendritic cells (%)** |
|---|---|---|---|
| Normal control group | 8 | 3.78±0.38 | 3.67±0.23 |
| Enteric placebo granule group | 7 | 14.20±1.60^{&&} | 22.32±1.45^{&&} |
| Ordinary lysozyme granule group | 8 | 13.12±1.22^{&&} | 25.59±2.13^{&&} |
| Lysozyme enteric granule group | 8 | **5.91±1.87^{&}**^{##}** | **4.09±0.45**^{##}** |

### Example 2 Effect of lysozyme enteric tablets on psoriasis for which halometasone and calcipotriol betamethasone were ineffective

A 48-year-old female patient had a 10-year history of psoriasis. The patient used halometasone cream and calcipotriol betamethasone ointment alternately; however, the effect was poor , and the PASI score was 6.8. Thereafter, she took the lysozyme enteric tablets twice a day, 100 mg each time, and after continuous use for 2 months, no significant improvement appeared and the PASI was 6.5. Thereafter, the administration was adjusted to taking lysozyme enteric tablets twice a day with 250 mg each time, and after continuous administration for 2 months, the PASI score was decreased by 52%. After further continued administration twice a day with 1000 mg each time for 3 months, the PASI score was decreased by 90%, the scales basically disappeared, and there was a small number of erythema. After further continued administration twice a day with 500 mg each time for 2 months, the skin lesions and erythema all disappeared and the skin returned to normal state, thus the drug administration was stopped. The patient was followed up one year later, and no symptoms of psoriasis was found. Three years later, the patient self-reported that she was in good condition and there was no recurrence of psoriasis. During this period, no other drugs for treating psoriasis were taken.

### Example 3 Effects of lysozyme buccal tablets and lysozyme enteric tablets on psoriasis for which calcipotriol was ineffective

A 58-year-old male patient with psoriasis had been ill for 4 years, and the main affected parts were arms, thighs, and lower legs. The patient used calcipotriol ointment for 1 month. The skin irritation was severe, and the condition had no sign of relief. The PASI score was 5.3, which was only 10% better than that before treatment. Thereafter, he took lysozyme buccal tablets twice a day with 500 mg each time, and after continuous use for 2 months, no alleviation appeared. After further administration twice a day with 1000 mg each time for 1 month, there was still no alleviation, so the drug administration was discontinued. Two years later, the patient was followed up, and he self-reported that after the drug withdrawal of lysozyme, he took methotrexate tablets and the psoriasis was relieved; however, he relapsed twice afterwards, and there were still a large number of erythema on the thighs. This patient was recommended to try the lysozyme enteric tablets. Thus the patient was advised to take the lysozyme enteric tablets three times a day with 1000 mg each time, and after continuous use for 6 months, the skin gradually returned to normal state.

### Example 4 Effect of lysozyme enteric tablets on psoriasis for which methotrexate was ineffective

A 41-year-old female patient had a 3-year history of psoriasis. She was healed after taking tretinoin cream, calcipotriol ointment and other treatments, however, she relapsed three months after the drug withdrawal. After an oral methotrexate treatment, the condition was not alleviated. The PASI score was 9.3, and desquamation and skin infiltration appeared in multiple areas of the front chest and back. Thereafter, she took the lysozyme enteric tablets three times a day with 2000 mg each time. After continuous use for 4 months, the condition was alleviated, the skin lesions and desquamation in the affected areas were significantly reduced, and the PASI was decreased by 60%. After further use for 3 months, only a small number of erythema remained, and the PASI was 1.7. The treatment was continued at a dose of 1000 mg twice a day for another 3 months, the skin returned to normal, and the drug administration was discontinued. Three years after the drug withdrawal, the patient was followed up and self-reported that no psoriasis relapse had occurred.

### Example 5 Effect of colonic enteric lysozyme tablets on psoriasis for which cyclosporine was ineffective

A 52-year-old female patient had a 6-year history of psoriasis. Two months of treatment with tazarotene cream was found ineffective, afterwards the patient was treated with cyclosporine capsules, and after 10 days, small pustules began to appear densely on multiple areas of the skin. She was diagnosed with pustular psoriasis, and the PASI score reached 11.7. Thereafter, she took the colonic enteric lysozyme tablets three times a day with 2000 mg each time. After continuous use for 2 months, a large area of pustules disappeared and the PASI was decreased by 85%. After continued use for another month, the pustules disappeared completely. After further continued administration at a dose of 1000 mg twice a day for 1 month, the skin returned to normal, and the drug administration was stopped. It had been five years since the drug withdrawal, and the patient had a normal diet and life with no recurrence of psoriasis.

### Example 6 Effect of colonic enteric lysozyme tablets on psoriasis for which TNF-α inhibitors were ineffective

A 37-year-old male had a 5-year history of psoriasis, with desquamation and skin infiltration in multiple areas of the front chest and back. He had used tretinoin cream, calcipotriol ointment, methotrexate tablets, and other treatments in the past. The condition was not alleviated and even turned to severe psoriasis with a PASI score of 13.8. Adalimumab was used for treatment, and after one course of treatment, the PASI was only decreased by 32%. Thereafter, he took the colonic enteric lysozyme tablets twice a day with 2000 mg each time. After continuous use for one and a half months, the condition was improved significantly, and it was hardly detected in the affected areas of the upper limbs. After further use for one month, the thighs, front chest and back were significantly ameliorated, and the PASI score was 1.1. The treatment was continued at a dose of 1000 mg twice a day for another month, the skin basically returned to normal, and the drug administration was discontinued. Three years after the drug withdrawal, the patient was followed up and self-reported that no psoriasis relapse had occurred.

### Example 7 Effect of colonic enteric lysozyme tablets on psoriasis for which IL-17 inhibitors were ineffective

A 62-year-old female patient had a 15-year history of psoriasis. In the last treatment, secukinumab was used; however, intolerance occurred. It was evaluated the PASI was decreased by only 10%, and the score was still as high as 8.7. Thereafter, she took the colonic enteric lysozyme tablets twice a day with 1000 mg each time. After continuous use for 2 months, the PASI was decreased to 2.0. The treatment was maintained at a dose of 500 mg twice a day for another 2 months, the skin of the patient returned to normal, so the drug administration was discontinued. Three years after the drug withdrawal, the patient was followed up and he self-reported that her body was healthy, and no psoriasis relapse had occurred.

The above case studies showed that for some patients with psoriasis for which existing drug treatments had been found ineffective, the PASI scores of these patients were generally not less than 5, and after using lysozyme preparations, they have unexpectedly obtained very good results. After long-term use, the disease was basically completely relieved. Critically, there had been no psoriasis relapse for several years since the drug withdrawal, and no adverse effects associated with lysozyme occurred. From these patients with drug-resistant psoriasis who took lysozyme preparations, it was observed that ordinary oral lysozyme preparations (such as buccal tablets) were less effective, whereas the enteric preparations (such as enteric tablets) had relatively good effects. In addition, the colonic enteric preparations (such as colonic enteric tablets) had the best effects and could take effect quickly.

## Claims

1. Lysozyme for use in the prevention of psoriasis, treatment of psoriasis, and/or prevention of psoriasis recurrence, wherein the PASI score of the psoriasis is not less than 5, wherein lysozyme is prepared into a medicament, and the medicament is an enteric preparation.

2. Lysozyme for use according to claim 1, wherein the PASI score of the psoriasis is not less than 8; and preferably, the psoriasis is drug-resistant psoriasis.

3. Lysozyme for use according to claim 2, wherein for the drug-resistant psoriasis, treatments with anti-inflammatory agents, anti-cell proliferation agents, and/or immunomodulators have been found ineffective.

4. Lysozyme for use according to claim 1 or 2, wherein the enteric preparation is a colonic enteric preparation.

5. Lysozyme for use according to claim 1, wherein lysozyme is prepared into a medicament, and the potency of lysozyme in the medicament is more than 20000 U/mg.

6. Lysozyme for use according to claim 5, wherein the dosage of lysozyme in the medicament is 0.3-20 g/day; preferably, the dosage of lysozyme in the medicament is 0.5-15 g/day; and more preferably, the dosage of lysozyme in the medicament is 1-6 g/day; furthermore, after the dosage of lysozyme in the medicament is determined, the medicament is administered 1-3 times a day.

7. A pharmaceutical composition for use in the prevention of psoriasis, the prevention of psoriasis recurrence or in treating psoriasis, wherein the composition comprises lysozyme, wherein the pharmaceutical composition is an enteric preparation.

8. The pharmaceutical composition for use according to claim 7, wherein the PASI score of the psoriasis is not less than 5; preferably, the PASI score of the psoriasis is not less than 8; and more preferably, the psoriasis is drug-resistant psoriasis.

9. The pharmaceutical composition for use according to claim 8, wherein for the drug-resistant psoriasis, treatments with anti-inflammatory agents, anti-cell proliferation agents, and/or immunomodulators have been found ineffective.

10. The pharmaceutical composition for use according to claim 7 or 8, wherein the enteric preparation is a colonic enteric preparation.

11. The pharmaceutical composition for use according to claim 10, wherein the potency of lysozyme in the pharmaceutical composition is more than 20000 U/mg.

12. The pharmaceutical composition for use according to claim 11, wherein the dosage of lysozyme in the pharmaceutical composition is 0.3-20 g/day; preferably, the dosage of lysozyme in the pharmaceutical composition is 0.5-15 g/day; and more preferably, the dosage lysozyme in of the pharmaceutical composition is 1-6 g/day; furthermore, after the dosage lysozyme in of the pharmaceutical composition is determined, the pharmaceutical composition is administered 1-3 times a day.

## Patentansprüche

1. Lysozym zur Verwendung bei der Vorbeugung von Schuppenflechte, der Behandlung von Schuppenflechte und/oder der Vorbeugung des Wiederauftretens von Schuppenflechte, wobei der PASI-Wert der Schuppenflechte nicht weniger als 5 beträgt, wobei Lysozym zu einem Arzneimittel hergestellt wird und das Arzneimittel eine magensaftresistente Zubereitung ist.

2. Lysozym zur Verwendung nach Anspruch 1, wobei der PASI-Wert der Psoriasis nicht weniger als 8 beträgt; und vorzugsweise ist die Psoriasis eine arzneimittelresistente Psoriasis.

3. Lysozym zur Verwendung nach Anspruch 2, wobei sich bei der Behandlung arzneimittelresistenter Psoriasis mit entzündungshemmenden Mitteln, Mittel gegen Zellproliferation und/oder Immunmodulatoren als unwirksam erwiesen haben.

4. Lysozym zur Verwendung nach Anspruch 1 oder 2, wobei die enterische Zubereitung eine kolonische enterische Zubereitung ist.

5. Lysozym zur Verwendung nach Anspruch 1, wobei Lysozym zu einem Arzneimittel hergestellt wird und Wirkstoffgehalt von Lysozym in dem Arzneimittel mehr als 20000 U/mg beträgt.

6. Lysozym zur Verwendung nach Anspruch 5, wobei die Lysozym-Dosierung in dem Arzneimittel 0,3-20 g/Tag beträgt; vorzugsweise beträgt die Lysozym-Dosierung in dem Arzneimittel 0,5-15 g/Tag; und noch bevorzugter beträgt die Lysozym-Dosierung in dem Arzneimittel 1-6 g/Tag; ferner wird das Arzneimittel, nachdem die Lysozym-Dosierung in dem Arzneimittel bestimmt wurde, 1-3 mal pro Tag verabreicht.

7. Pharmazeutische Zusammensetzung zur Verwendung bei der Vorbeugung von Psoriasis, der Vorbeugung des Wiederauftretens von Psoriasis oder bei der Behandlung von Psoriasis, wobei die Zusammensetzung Lysozym umfasst, wobei die pharmazeutische Zusammensetzung eine enterische Zubereitung ist.

8. Pharmazeutische Zusammensetzung zur Verwendung nach Anspruch 7, wobei der PASI-Wert der Psoriasis nicht weniger als 5 beträgt; vorzugsweise beträgt der PASI-Wert der Psoriasis nicht weniger als 8; und noch bevorzugter ist die Psoriasis eine arzneimittelresistente Psoriasis.

9. Pharmazeutische Zusammensetzung zur Verwendung gemäß Anspruch 8, wobei sich bei der arzneimittelresistenten Psoriasis Behandlungen mit entzündungshemmenden Mitteln, Mitteln gegen Zellproliferation und/oder Immunmodulatoren als unwirksam erwiesen haben.

10. Pharmazeutische Zusammensetzung zur Verwendung nach Anspruch 7 oder 8, wobei das enterische Präparat ein kolonisches enterisches Präparat ist.

11. Pharmazeutische Zusammensetzung zur Verwendung gemäß Anspruch 10, wobei der Wirkstoffgehalt von Lysozym in der pharmazeutischen Zusammensetzung mehr als 20000 U/mg beträgt.

12. Pharmazeutische Zusammensetzung zur Verwendung gemäß Anspruch 11, wobei die Lysozym-Dosierung in der pharmazeutischen Zusammensetzung 0,3-20 g/Tag beträgt; vorzugsweise beträgt die Lysozym-Dosierung in der pharmazeutischen Zusammensetzung 0,5-15 g/Tag; und noch bevorzugter beträgt die Lysozym-Dosierung in der pharmazeutischen Zusammensetzung 1-6 g/Tag; ferner , nachdem die Lysozym-Dosierung in der pharmazeutischen Zusammensetzung bestimmt wurde, wird die pharmazeutische Zusammensetzung 1-3 mal pro Tag verabreicht.

## Revendications

1. Lysozyme pour la prévention du psoriasis, le traitement du psoriasis et/ou la prévention de la récurrence du psoriasis, dans lequel le score PASI du psoriasis n'est pas inférieur à 5, dans lequel le lysozyme est préparé dans un médicament, et le médicament est une préparation entérique.

2. Lysozyme à utiliser selon la revendication 1, dans lequel le score PASI du psoriasis n'est pas inférieur à 8 ; de préférence, le psoriasis est un psoriasis résistant aux médicaments.

3. Lysozyme utilisé selon la revendication 2, dans lequel, pour le psoriasis résistant aux médicaments, les traitements à base d'agents anti-inflammatoires, d'agents de prolifération cellulaire et/ou d'immunomodulateurs se sont révélés inefficaces.

4. Lysozyme à utiliser selon la revendication 1 ou 2, dans lequel la préparation entérique est une préparation entérique colonique.

5. Lysozyme à utiliser selon la revendication 1, dans lequel le lysozyme est préparé dans un médicament, et la puissance du lysozyme dans le médicament est supérieure à 20000 U/mg.

6. Lysozyme à utiliser selon la revendication 5, dans lequel le dosage de lysozyme dans le médicament est de 0,3-20 g/jour ; de préférence, le dosage de lysozyme dans le médicament est de 0,5-15 g/jour ; et plus préférablement, le dosage de lysozyme dans le médicament est de 1-6 g/jour ; en outre, après que le dosage de lysozyme dans le médicament a été déterminé, le médicament est administré 1-3 fois par jour.

7. Composition pharmaceutique destinée à la prévention du psoriasis, à la prévention de la récurrence du psoriasis ou au traitement du psoriasis, dans laquelle la composition comprend du lysozyme, la composition pharmaceutique étant une préparation entérique.

8. Composition pharmaceutique à utiliser selon la revendication 7, dans laquelle le score PASI du psoriasis n'est pas inférieur à 5 ; de préférence, le score PASI du psoriasis n'est pas inférieur à 8 ; et de préférence encore, le psoriasis est un psoriasis résistant aux médicaments.

9. Composition pharmaceutique à utiliser selon la revendication 8, dans laquelle, pour le psoriasis résistant aux médicaments, les traitements à base d'agents anti-inflammatoires, d'agents de prolifération cellulaire et/ou d'immunomodulateurs se sont révélés inefficaces.

10. Composition pharmaceutique à utiliser selon la revendication 7 ou 8, dans laquelle la préparation entérique est une préparation entérique colonique.

11. Composition pharmaceutique à utiliser selon la revendication 10, dans laquelle la puissance du lysozyme dans la composition pharmaceutique est supérieure à 20000 U/mg.

12. Composition pharmaceutique à utiliser selon la revendication 11, dans laquelle le dosage de lysozyme dans la composition pharmaceutique est de 0,3-20 g/jour ; de préférence, le dosage de lysozyme dans la composition pharmaceutique est de 0,5-15 g/jour ; et plus préférablement, le dosage de lysozyme dans la composition pharmaceutique est de 1-6 g/jour ; en outre, après que le dosage de lysozyme dans la composition pharmaceutique a été déterminé, la composition pharmaceutique est administrée 1-3 fois s par jour.
